(19) Europäisches Patentamt European Patent Office Office européen des brevets

(11) **EP 4 183 321 A1**

(12) **EUROPEAN PATENT APPLICATION**

(43) Date of publication:
**24.05.2023 Bulletin 2023/21**

(21) Application number: **22207401.5**

(22) Date of filing: **15.11.2022**

(51) International Patent Classification (IPC):
**A61B 3/10** (2006.01)

(52) Cooperative Patent Classification (CPC):
**A61B 3/102; A61B 3/1005**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2021 JP 2021186837**

(71) Applicant: **Tomey Corporation**
**Nagoya-shi, Aichi-ken, 451-0051 (JP)**

(72) Inventor: **Kamo, Takashi**
**Aichi-ken, 451-0051 (JP)**

(74) Representative: **BSB Patentanwälte**
**Schütte & Engelen Partnerschaft mbB**
**Am Markt 10**
**59302 Oelde (DE)**

(54) **SD-OCT DEVICE**

(57) An SD-OCT device includes a light source that outputs light including wavelength components; a branching unit that branches, from the light output from the light source, at least reference light following a reference optical path and measurement light applied to a measurement target; a transmission unit that transmits interference light between the reference light and the measurement light returning from the measurement target; a light receiving unit having linearly arranged light receiving elements; and an optical system that disperses the interference light output from the transmission unit and condenses the interference light on the light receiving unit for each wavelength component. A diameter of the wavelength component having a predetermined wavelength, which is condensed on the light receiving unit by the optical system, is equal to or less than an average wavelength of a wavy signal detected via the light receiving unit upon reception of the interference light.

Fig. 2

**Description**

CROSS-REFERENCE TO RELATED APPLICATION

This application claims benefit of priority to Japanese Patent Application No. 2021-186837, filed November 17, 2021, the entire content of which is incorporated herein by reference.

BACKGROUND

Technical Field:

The present disclosure relates to an SD-OCT device.

Background Art:

Optical coherence tomography (OCT) is known as a technique for measuring a position of a measurement target using optical coherence. In the OCT, a structure of the measurement target is measured by utilizing interference between light returning from the measurement target after the measurement target is irradiated with light and reference light passing through a reference optical path. The OCT includes a system called Spectral Domain-OCT (SD-OCT) in which interference light between light returning from the measurement target and reference light is dispersed for each wavelength component, and the position of the measurement target is measured using the dispersed interference light. JP 2016-32578 A discloses a technique applicable to the SD-OCT.

JP 2016-32578 A discloses a configuration in which an optical path length of reference light is changed by moving a position of a mirror that reflects the reference light.

SUMMARY

The SD-OCT involves a problem that measurement accuracy is insufficient depending on the design of optical parts. Accordingly, the present disclosure provides an SD-OCT to suppress a decrease in accuracy in measurement by the SD-OCT.

An SD-OCT device according to the present disclosure includes a light source that outputs light including a plurality of wavelength components; a branching unit that branches, from the light output from the light source, at least reference light following a reference optical path and measurement light applied to a measurement target; a transmission unit that transmits interference light between the reference light and the measurement light returning from the measurement target; a light receiving unit in which a plurality of light receiving elements are linearly arranged; and an optical system that disperses the interference light output from the transmission unit and condenses the interference light on the light receiving unit for each of the wavelength components. A diameter of the wavelength component having a predetermined wavelength, which is condensed on the light receiving unit by the optical system, is equal to or less than an average wavelength of a wavy signal detected via the light receiving unit upon reception of the interference light.

That is, in the SD-OCT device, the diameter of the wavelength component having the predetermined wavelength, which is condensed on the light receiving unit, is equal to or less than the average wavelength of the wavy signal detected via the light receiving unit. Thus, overlapping of light beams of the wavelength components on the light receiving unit is reduced. When the wavelength components overlap with each other, signals of the wavelength components cannot be distinguished from each other in the overlapping portion, resulting in a decrease in accuracy of the signals detected via the light receiving unit. By suppressing such a circumstance, the SD-OCT device can suppress a decrease in accuracy in measurement by the SD-OCT.

BRIEF DESCRIPTION OF THE DRAWINGS

FIG. 1 is a diagram illustrating a configuration of an SD-OCT device according to an embodiment of the present disclosure;

FIG. 2 is a diagram illustrating the configuration of the SD-OCT device according to the embodiment of the present disclosure;

FIG. 3 is a diagram for explaining reception of interference light in a light receiving unit;

FIG. 4 is a diagram for explaining a detection signal detected by the light receiving unit;

FIG. 5 is a diagram for explaining a spot diameter of a wavelength component received by the light receiving unit;

FIG. 6 is a diagram for explaining overlapping of wavelength components; and
FIG. 7 is a flowchart illustrating an example of eye axis length measurement processing.

DETAILED DESCRIPTION

An example of an embodiment of the present disclosure will be described in the following order.

(1) Configuration of SD-OCT device:
(2) Eye axis length measurement processing:
(3) Other embodiments:

(1) Configuration of SD-OCT device:

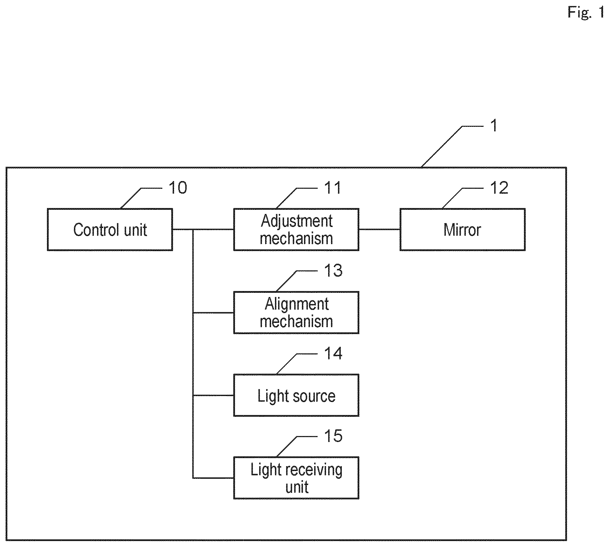

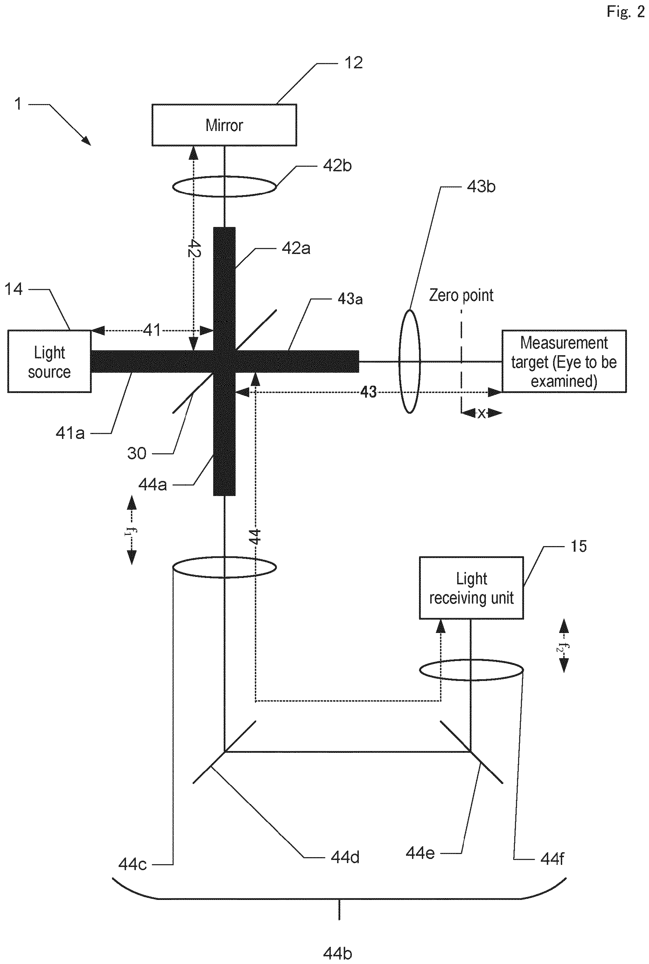

Hereinafter, an SD-OCT device 1 according to the present embodiment will be described. The SD-OCT device 1 of the present embodiment measures a position of a measurement target by the SD-OCT method using a corneal apex and fundus (retina) in an eyeball (hereinafter, eye to be examined) of a subject as the measurement target, and measures an eye axis length of the eye to be examined. FIGS. 1 and 2 are diagrams schematically illustrating a configuration of the SD-OCT device 1 of the present embodiment. The SD-OCT device 1 includes a control unit 10, an adjustment mechanism 11, a mirror 12, an alignment mechanism 13, a light source 14, and a light receiving unit 15. In addition, the SD-OCT device 1 includes optical members (a branching unit 30, transmission units 41a, 42a, 43a, and 44a, collimators 42b and 43b, and an optical system 44b) that form optical paths 41 to 44 of light output from the light source 14.

The control unit 10 includes a processor, a RAM, a ROM, and the like, and controls the SD-OCT device 1 by executing a program recorded in the ROM or the like. The adjustment mechanism 11 is a mechanism capable of moving the mirror 12 in a linear direction along the optical path 42. In the present embodiment, the adjustment mechanism 11 is a ball screw mechanism that moves the mirror 12, but may be another mechanism such as a slider crank mechanism or a power transmission mechanism such as a cam. The mirror 12 reflects the incident light. The control unit 10 adjusts a position of the mirror 12 via the adjustment mechanism 11. The alignment mechanism 13 is a mechanism used for adjusting a positional relationship between the SD-OCT device 1 and the measurement target. Before the measurement of the measurement target by the SD-OCT, the control unit 10 detects a position of the corneal apex of the eye to be examined of the subject present at a predetermined position via the alignment mechanism 13, and adjusts a position of the SD-OCT device 1 such that the detected position of the corneal apex and the SD-OCT device 1 have a predetermined positional relationship. The light source 14 outputs light in a predetermined wavelength band in response to an instruction from the control unit 10. In the present embodiment, the light source 14 outputs light in a wavelength band with a full width at half maximum of 60 nm centered on 840 nm. Hereinafter, a wavelength of a wavelength component defined as a wavelength component mainly contributing to measurement by the SD-OCT among wavelength components included in the light output from the light source 14 is defined as center wavelength. In the present embodiment, the center wavelength is 840 nm, which is a wavelength of the center in the wavelength band of the light output from the light source 14. Hereinafter, the center wavelength is denoted as $\lambda_0$. The light receiving unit 15 is a plurality of light receiving elements arranged linearly. In the present embodiment, the light receiving unit 15 is a sensor in which 2048 light receiving elements having a width of 7 $\mu$m are arranged, and has a width of 7 $\mu$m $\times$ 2048 = 14.336 mm.

The branching unit 30 is an optical member that branches reference light following reference optical path and measurement light applied to the measurement target from the light output from the light source 14, and can be configured by, for example, a filter coupler or the like. The transmission unit 41a is used to form the optical path 41, and is an optical fiber that transmits the light from the light source 14 to the branching unit 30 in the present embodiment. The optical path 41 is an optical path that causes the light output from the light source 14 to travel to the branching unit 30. Furthermore, the transmission unit 42a and the collimator 42b are used to form the optical path 42. The optical path 42 is an optical path through which the reference light branched by the branching unit 30 travels toward the mirror 12, and is also an optical path through which the reference light reflected by the mirror 12 and traveling in an opposite direction travels toward the branching unit 30. The transmission unit 42a is an optical fiber that transmits the reference light branched by the branching unit 30. The collimator 42b converts the light output from the transmission unit 42a into parallel light. Furthermore, the transmission unit 43a and the collimator 43b are used to form the optical path 43. The optical path 43 is an optical path through which the measurement light branched by the branching unit 30 travels toward the measurement target, and is also an optical path through which the measurement light returning from the measurement target travels toward the branching unit 30. The transmission unit 43a is an optical fiber that transmits the measurement light branched by the branching unit 30. The collimator 43b converts the light output from the transmission unit 43a into parallel light.

Furthermore, the transmission unit 44a and the optical system 44b are used to form the optical path 44. The optical path 44 is an optical path through which the interference light between the measurement light and the reference light generated by the branching unit 30 travels toward the light receiving unit 15. The transmission unit 44a is an optical fiber that transmits the interference light generated by the branching unit 30. The optical system 44b disperses the interference light output from the transmission unit 44a and condenses the interference light on the light receiving unit 15 for each of the wavelength components. The optical system 44b includes a lens 44c, a dispersion member 44d, a dispersion member 44e, and a lens 44f. The lens 44c is disposed on the optical path 44 at a position separated from an output end of the transmission unit 44a by a focal length fi of the lens 44c. Therefore, the lens 44c converts the interference light output from the transmission unit 44a and traveling while radially spreading around an optical axis into parallel light. Each of the dispersion members 44d and 44e disperses the incident light. In the present embodiment, each of the dispersion members 44d and 44e is a diffraction grating, but may be another optical member such as a prism. The dispersion members 44d and 44e of the present embodiment are diffraction gratings provided with 1800 slits per mm, but may be diffraction gratings provided with another number (for example, 2400 or the like) of slits per mm. A traveling direction of the interference light changes for each of the wavelength components due to dispersion by the dispersion members 44d and 44e. The lens 44f is disposed on the optical path 44 at a position separated from the light receiving unit 15 by a focal length $f_2$ of the lens 44f. The light receiving unit 15 is disposed to face the lens 44f, and is also disposed such that the plurality of light receiving elements of the light receiving unit 15 are arranged along a direction perpendicular to the optical axis of the lens 44f and perpendicular to the respective slits of the dispersion members 44d and 44e.

In the present embodiment, the SD-OCT device 1 generates the interference light from the light output from the light source 14 using a Michelson interferometer. The optical paths of the light output from the light source 14 in the SD-OCT device 1 will be described with reference to FIG. 2.

The light output from the light source 14 is transmitted through the transmission unit 41a of the optical path 41 and reaches the branching unit 30. The branching unit 30 branches the reference light and the measurement light from the reached light. Then, the branching unit 30 causes the reference light to travel to the optical path 42 and causes the measurement light to travel to the optical path 43.

The reference light that has traveled to the optical path 42 is transmitted through the transmission unit 42a, output from the transmission unit 42a, and reaches the mirror 12 via the collimator 42b. The reference light reflected by the mirror 12 travels through the optical path 42 again and reaches the branching unit 30 via the collimator 42b. The measurement light that has traveled from the branching unit 30 to the optical path 43 is transmitted through the transmission unit 43a, output from the transmission unit 43a, and reaches the measurement target via the collimator 43b. Then, reflection or scattering of the measurement light occurs in the measurement target. Thus, at least a part of the reflected or scattered measurement light travels in a direction opposite to an incident direction and thus returns from the measurement target. The measurement light returning from the measurement target travels through the optical path 43 again and reaches the branching unit 30 via the collimator 43b. The branching unit 30 combines the reference light and measurement light that has reached the branching unit 30 to generate the interference light between the reference light and the measurement light, and causes the generated interference light to travel to the optical path 44.

As described above, in the present embodiment, the reference light branched by the branching unit 30 travels through the optical path 42, the mirror 12, the optical path 42, the branching unit 30, and the optical path 44 in this order, and reaches the light receiving unit 15. Therefore, an optical path of the reference light is formed by the optical path 42, the mirror 12, and the optical path 44. Hereinafter, the optical path of the reference light is referred to as reference arm. The control unit 10 adjusts an optical path length of the reference arm by moving the mirror 12 via the adjustment mechanism 11. Here, the optical path length is a length of the optical path after conversion when the optical path is converted into an optical path in an air medium. For example, an optical path length of an optical path having a length of 1 m in a medium having a refractive index with respect to air of 1.2 is refractive index of the medium of 1.2 × actual length of 1 m = 1.2 m.

The measurement light branched by the branching unit travels through the optical path 43, the measurement target, the optical path 43, the branching unit 30, and the optical path 44 in this order, and reaches the light receiving unit 15. Therefore, in the present embodiment, the optical path of the measurement light is formed by the optical path 43, the measurement target, and the optical path 44. Hereinafter, an optical path of the measurement light is referred to as measurement arm.

In the present embodiment, the control unit 10 switches the reference arm for measuring the corneal apex of the eye to be examined and the reference arm for measuring the retina of the eye to be examined by moving the mirror 12 via the adjustment mechanism 11. Hereinafter, the reference arm for measuring the corneal apex of the eye to be examined is referred to as reference arm for the cornea. In addition, the reference arm for measuring the retina of the eye to be examined is referred to as reference arm for the retina. In the present embodiment, the zero point in the measurement arm in a case where the reference arm for the cornea is used is adjusted to be located at a position in the vicinity of the corneal apex of the eye to be examined aligned in the optical path 43 and in front of the corneal apex. Here, the front is front as viewed from the subject. Also hereinafter, the front indicates the front as viewed from the subject. Here, the zero point is a position on the measurement arm, and is a position where the optical path length of the measurement light in a case where the measurement light is reflected back in the opposite direction at that position is the same as the optical path length of the reference arm. In addition, when the reference arm for the retina is used, the zero point in the measurement arm is adjusted to a position on the optical path 43 at a predetermined distance behind the cornea of the eye to be examined. In the present embodiment, the predetermined distance is a minimum value of the length assumed as the eye axis length of the eyeball.

The interference light traveling through the optical path 44 becomes parallel light via the lens 44c and reaches the dispersion member 44d. The interference light that has reached the dispersion member 44d is dispersed to be divided for each of the wavelength components, and reaches the dispersion member 44e. Then, the interference light that has reached the dispersion member 44e is dispersed again and reaches the lens 44f. The interference light that has reached the lens 44f for each of the wavelength components is divided for each of the wavelength components, travels through an optical path different for each of the wavelength components, and is condensed on the light receiving unit 15. Therefore, on the light receiving unit 15, the interference light is condensed at different positions for the respective wavelength components. Thus, the control unit 10 can detect intensity of each of the wavelength components of the dispersed interference light via the plurality of light receiving elements of the light receiving unit 15.

Note that the wavelength of light that can be received by the light receiving unit 15 is obtained in advance from a positional relationship between the light receiving unit 15 and the dispersion member 44e. Hereinafter, the smallest wavelength of the light that can be received by the light receiving unit 15 is denoted as $\lambda_1$. The maximum wavelength of the light that can be received by the light receiving unit 15 is denoted as $\lambda_2$.

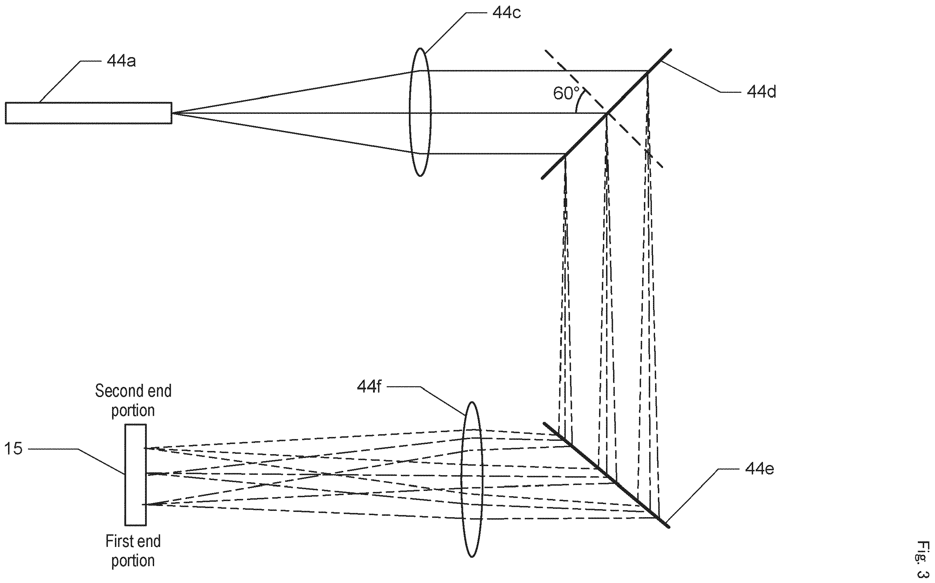

Here, a situation where the light output from the transmission unit 44a in the optical path 44 is condensed on the light receiving unit 15 will be described with reference to FIG. 3.

When output from the transmission unit 44a, the interference light enters the lens 44c while radially spreading around the optical axis. The lens 44c converts the incident interference light into parallel light. The interference light converted into the parallel light by the lens 44c is incident on the dispersion member 44d. In the present embodiment, the optical system 44b is designed such that the interference light converted into the parallel light by the lens 44c is incident on the dispersion member 44d at an incident angle of 60°. However, the optical system 44b may be designed such that the interference light converted into the parallel light by the lens 44c is incident on the dispersion member 44d at another incident angle. In the dispersion member 44d, the interference light is dispersed and divided into components having different wavelengths. In FIG. 3, as an example, optical paths of wavelength components of three wavelengths are shown by a broken line, a dashed line, and a double-dashed line, respectively. The interference light dispersed by the dispersion member 44d is parallel light when viewed for each of the wavelength components.

The interference light dispersed by the dispersion member 44d is incident on the dispersion member 44e. In the dispersion member 44e, the interference light is further dispersed. The interference light dispersed by the dispersion member 44e is parallel light when viewed for each of the wavelength components. The interference light dispersed by the dispersion member 44e is incident on the lens 44f. The interference light dispersed by the dispersion member 44e is parallel light for each of the wavelength components, and the parallel light for each of the wavelength components is incident on the lens 44f. Therefore, the respective wavelength components of the interference light are condensed at different positions on the light receiving unit 15 disposed at a position separated from the lens 44f by the focal length $f_2$.

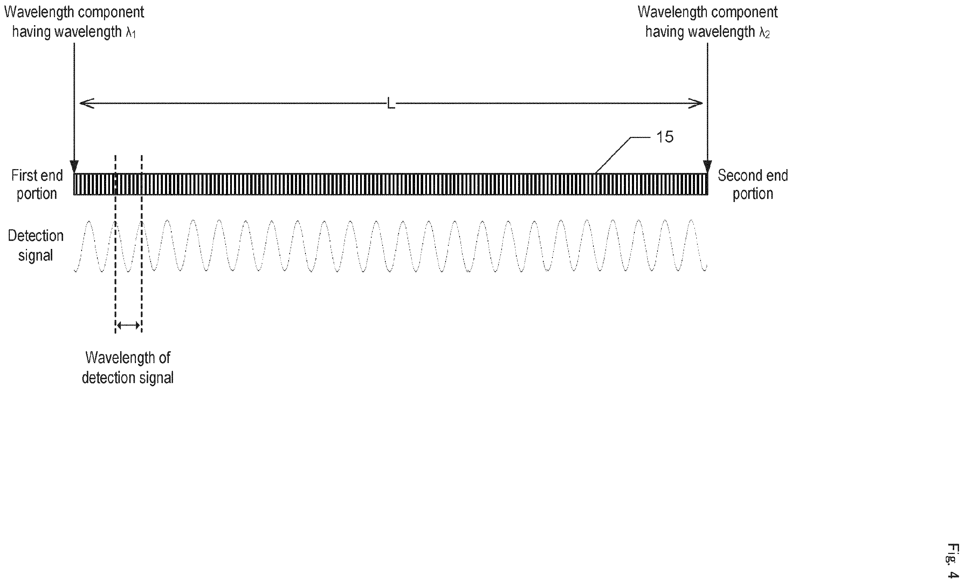

Here, a signal detected via the light receiving unit 15 will be described with reference to FIG. 4.

At one end portion (hereinafter referred to as first end portion) of the light receiving unit 15, a wavelength component having the wavelength $\lambda_1$ in the interference light is condensed. At the other end portion (hereinafter referred to as second end portion) of the light receiving unit 15, a wavelength component having the wavelength $\lambda_2$ in the interference light is condensed. Furthermore, the wavelength of the condensed light continuously varies in a region from the first end portion to the second end portion, in the light receiving unit 15. A wavelength component having a shorter wavelength is condensed at a position closer to the first end portion on the light receiving unit 15, and a wavelength component having a longer wavelength is condensed at a position closer to the second end portion on the light receiving unit 15. The control unit 10 detects a signal in which the position (distance from the first end portion) of each of the light receiving elements of the light receiving unit 15 on the light receiving unit 15 is associated with a signal of intensity of the light detected via each of the light receiving elements of the light receiving unit 15. Hereinafter, the signal detected here (signal in which the position of each of the light receiving elements of the light receiving unit 15 on the light receiving unit 15 is associated with the signal of the intensity of light detected via each of the light receiving elements of the light receiving unit 15) is referred to as detection signal.

What signal is detected as the detection signal will be described. The intensity of the wavelength component having the wavelength $\lambda$ in the interference light is expressed by Formula 1 below. [Mathematical Formula 1]

$$A \times cos(4\pi x/\lambda) \qquad (1)$$

In Formula 1, A represents a constant. x represents an optical path length of an optical path from the zero point to the measurement target in the measurement arm. Therefore, 2x represents a difference in optical path length between the reference light and the measurement light (a difference in optical path length between the reference arm and the measurement arm). Since the wavelength component having the wavelength $\lambda_1$ is condensed at the first end portion of the light receiving unit 15, a signal having a phase of $(4\pi x/\lambda_1)$ is detected at the first end portion. Furthermore, since the wavelength component having the wavelength $\lambda_2$ is condensed at the second end portion of the light receiving unit 15, a signal having a phase of $(4\pi x/\lambda_2)$ is detected at the second end portion. In the light receiving unit 15, the wavelength of the condensed light continuously varies in a region from the first end portion to the second end portion. Therefore, as illustrated in FIG. 4, the detection signal detected by the light receiving unit 15 is a wavy signal whose phase continuously varies from $(4\pi|x|/\lambda_1)$ to $(4\pi|x|/k_2)$, in a region from the first end portion to the second end portion. A number of waves included in the wavy detection signal is obtained, as in Equation (2) below, by dividing a value of phase variation from the first end portion to the second end portion by $2\pi$.
[Mathematical Formula 2]

$$((4\pi|x|/\lambda_1) - (4\pi|x|/\lambda_2))/(2\pi) = 2|x|(1/\lambda_1 - 1/\lambda_2) \qquad (2)$$

Therefore, when the length of the light receiving unit 15 is L, an average wavelength wl of the detection signal is obtained, as in Equation (3), by dividing L by the number obtained as in Equation (2).
[Mathematical Formula 3]

$$wl = L/(2|x|(1/\lambda_1 - 1/\lambda_2)) \qquad (3)$$

As shown in Equations (2) and (3), as the optical path length x from the zero point to the measurement target in the measurement arm increases (as the difference 2x in optical path length between the reference arm and the measurement arm increases), the number of waves in the detection signal increases, and the average wavelength wl of the detection signal decreases.

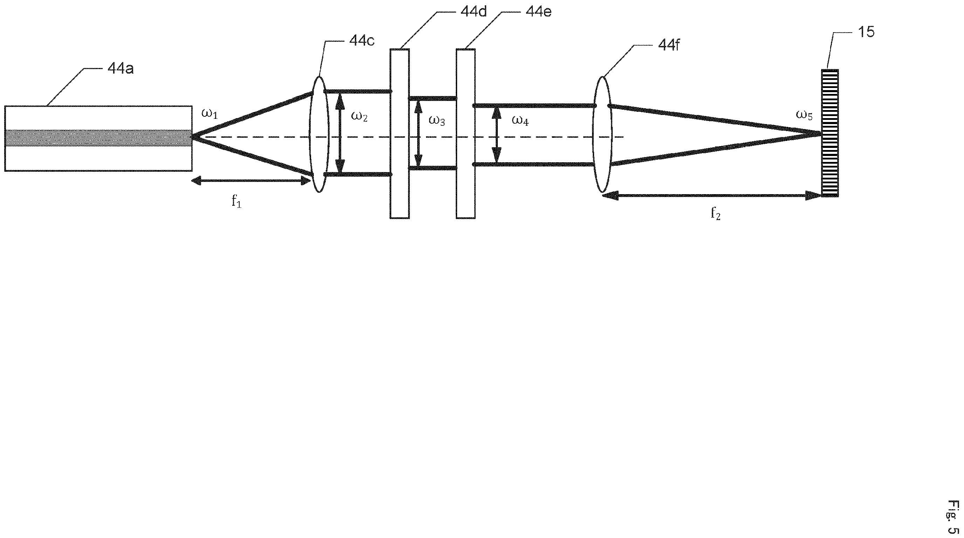

Subsequently, a size of a condensing portion of each of the wavelength components of the interference light condensed on the light receiving unit 15 will be described with reference to FIG. 5. No lens can be used to condense light as a complete point. Therefore, each of the wavelength components of the interference light is condensed in a spot shape having a finite size on the light receiving unit 15. Hereinafter, a diameter of the condensing portion of the wavelength component of the interference light on the light receiving unit 15, that is, a diameter in a direction parallel to the direction in which the plurality of light receiving elements of the light receiving unit 15 are arranged is defined as spot diameter.

A diameter of the wavelength component having the wavelength $\lambda$ of the interference light output from the transmission unit 44a, which is an optical fiber, is a Mode Field Diameter (MFD) in a case where the light having the wavelength $\lambda$ is output from the transmission unit 44a. The MFD is a value defined as diameter of light output from an optical fiber. The light output from the optical fiber spreads circularly around the optical axis in a Gaussian distribution. The MFD is a diameter in a circular region including 86.5% of the total energy of light around the optical axis in the spread of the Gaussian distribution. The MFD in a case where the light having the wavelength $\lambda$ is output from the transmission unit 44a is obtained in advance from the wavelength $\lambda$ of the transmitted light, a diameter of a core of the transmission unit 44a, and refractive indexes of the core and a clad of the transmission unit 44a. Hereinafter, the diameter of the wavelength component having the wavelength $\lambda$ in the interference light output from the transmission unit 44a is defined as $\omega_1$. $\omega_1$ is an MFD in a case where the light having the wavelength $\lambda$ is output from the transmission unit 44a.

The light travels, and thus the diameter increases due to a diffraction phenomenon. When light having a diameter $\omega_0$ travels by a distance z, the diameter of the light is as shown in Equation (4) below.
[Mathematical Formula 4]

$$\omega_0\sqrt{1 + (\lambda z/(\pi\omega_0{}^2))^2} \approx \lambda z/(\pi\omega_0) \qquad (4)$$

Therefore, when the light of the diameter $\omega_1$ and the wavelength $\lambda$ output from the transmission unit 44a travels by the focal length fi and reaches the lens 44c, the diameter becomes $\lambda f_1/(\pi\omega_1)$. Hereinafter, a diameter when the light having the diameter $\omega_1$ and the wavelength $\lambda$ travels by the focal length fi and reaches the lens 44c is defined as $\omega_2$. That is, $\omega_2 = \lambda f_1/(\pi\omega_1)$.

The light that has penetrated the lens 44c becomes parallel light, and thus enters the dispersion member 44d with a constant diameter.

Here, a case where light enters a diffraction grating will be described. In a case where light is incident on the diffraction grating at an incident angle $\alpha$ and is emitted at a diffraction angle $\beta$, a relationship represented by Equation (5) below is satisfied. In Equation (5), d represents a slit interval in each of the dispersion members 44d and 44e. Also, in Equation (5), m represents a diffraction order and is an arbitrary integer.
[Mathematical Formula 5]

$$d\,sin(\alpha) + d\,sin(\beta) = m\lambda \qquad (5)$$

In addition, the diameter of the light that has passed through the diffraction grating, in a direction perpendicular to the slit of the diffraction grating through which the light has passed, changes. The diameter of the light in the direction perpendicular to the slit of the diffraction grating after passing is cos $(\beta)$/cos $(\alpha)$ times the diameter before passing. Hereinafter, the diameter of the light traveling through the optical path 44 in the direction perpendicular to the slit of the diffraction grating as each of the dispersion members 44d and 44e is referred to as beam diameter. Therefore, when the incident angle when the light having the wavelength $\lambda$ enters the dispersion member 44d is $\alpha_1$ and the diffraction angle is $\beta_1$, in a case where the light passes through the dispersion member 44d, a beam diameter $\omega_3$ of the light is expressed by Equation (6) below. As described above, in the present embodiment, the optical system 44b is designed such that the incident angle when the light that has passed through the lens 44c and become parallel light enters the dispersion member 44d is 60°.
[Mathematical Formula 6]

$$\omega_3 = (\lambda f_1/(\pi\omega_1)) \times (cos(\beta_1)/cos(\alpha_1)) \qquad (6)$$

The light having the wavelength $\lambda$ that has passed through the dispersion member 44d is incident on the dispersion member 44e as the parallel light. When the incident angle when the light having the wavelength $\lambda$ enters the dispersion member 44e is $\alpha_2$ and the diffraction angle is $\beta_2$, in a case where the light passes through the dispersion member 44e, a beam diameter $\omega_4$ of the light is expressed by Equation (7) below.
[Mathematical Formula 7]

$$\omega_4 = (\lambda f_1/(\pi\omega_1)) \times (cos(\beta_1)/cos(\alpha_1)) \times (cos(\beta_2)/cos(\alpha_2)) \qquad (7)$$

Since the light of the wavelength $\lambda$ that has passed through the dispersion member 44e is parallel light, the light enters the lens 44f with a constant diameter.
Here, in the optical system 44b, when a number of diffraction gratings included between the lens 44c and the lens 44f is n, and the incident angle and diffraction angle of the light having the wavelength $\lambda$ in an $i^{th}$ diffraction grating are $\alpha_i$ and $\beta_i$, respectively, the diameter of the light of the wavelength $\lambda$ incident on the lens 44f can be generalized as in Equation (8) below.
[Mathematical Formula 8]

$$\omega_4 = (\lambda f_1/(\pi\omega_1)) \times \prod_{i=1}^{n}(cos(\beta_i)/cos(\alpha_i)) \qquad (8)$$

The light of the wavelength $\lambda$ incident on the lens 44f is condensed on the light receiving unit 15. A spot diameter of the light of the wavelength $\lambda$ condensed on the light receiving unit 15 is defined as $\omega_5$. The beam diameter $\omega_4$ of the light of the wavelength $\lambda$ incident on the lens 44f is expressed by Equation (8). In this case, n in Equation (8) is 2. In addition, the beam diameter is equal to $\lambda f_2/(\pi\omega_5)$, which is a diameter when the light having the diameter cos travels from the light receiving unit 15 to the lens 44f by the focal length $f_2$. Therefore, a relationship of Equation (9) below is established.
[Mathematical Formula 9]

$$(\lambda f_1/(\pi\omega_1)) \times \prod_{i=1}^{n}(cos(\beta_i)/cos(\alpha_i)) = \lambda f_2/(\pi\omega_5) \qquad (9)$$

From Equation (9), the spot diameter cos when the light having the wavelength $\lambda$ is condensed on the light receiving unit 15 is expressed by Equation (10) below. Power in Equation (10) is a magnification of the optical system 44b. The magnification Power of the optical system 44b is expressed by (focal length $f_2$ of lens 44f)/(focal length fi of lens 44c). In addition, n in Equation (10) is 2 since the optical system 44b includes the two dispersion members 44d and 44e in the present embodiment.

[Mathematical Formula 10]

$$\omega_5 = \omega_1 \times Power \times \prod_{i=1}^{n}(cos(\alpha_i)/cos(\beta_i)) \qquad (1\,0)$$

As described above, the wavelength component having the wavelength $\lambda$ in the interference light is condensed on the light receiving unit 15 with a size of the spot diameter $\omega_5$.

The SD-OCT device 1 of the present embodiment is designed such that the spot diameter of the wavelength component of the predetermined wavelength in the interference light condensed on the light receiving unit 15 is equal to or less than the average wavelength of the detection signal detected via the light receiving unit 15. This will be described in more detail below. In the present embodiment, the predetermined wavelength is the center wavelength $\lambda_0$. Furthermore, the intention of such design will be described later.

The spot diameter of the wavelength component having the center wavelength $\lambda_0$ in the interference light condensed on the light receiving unit 15 is obtained as follows. The MFD in a case where light having the center wavelength $\lambda_0$ is output from the transmission unit 44a, which is an optical fiber, is obtained in advance from the center wavelength $\lambda_0$, the diameter of the core of the transmission unit 44a, and the refractive indexes of the core and clad of the transmission unit 44a. As described above, the incident angle when the wavelength component having the center wavelength $\lambda_0$ converted into parallel light by the lens 44c is incident on the dispersion member 44d is determined in advance and is 60°. In addition, the diffraction angle when the wavelength component having the center wavelength $\lambda_0$ is incident on the dispersion member 44d at an incident angle of 60° is obtained as follows. Specifically, an equation for the diffraction angle $\beta$ is obtained by substituting this incident angle of 60° for $\alpha$, substituting the slit interval of the dispersion member 44d for d, substituting the center wavelength $\lambda_0$ for $\lambda$, and substituting a predetermined order for m, in Equation (5). By solving this equation, the diffraction angle when the wavelength component having the center wavelength $\lambda_0$ is incident on the dispersion member 44d at an incident angle of 60° is obtained as $\beta$.

In addition, from the diffraction angle obtained here and the positional relationship between the dispersion member 44d and the dispersion member 44e, the incident angle when the wavelength component having the center wavelength $\lambda_0$ is incident on the dispersion member 44e is obtained. In addition, the diffraction angle when the wavelength component having the center wavelength $\lambda_0$ is incident on the dispersion member 44e at the obtained incident angle is obtained as follows. Specifically, an equation for the diffraction angle $\beta$ is obtained by substituting this incident angle for $\alpha$, substituting the slit interval of the dispersion member 44e for d, substituting the center wavelength $\lambda_0$ for $\lambda$, and substituting the predetermined order for m, in Equation (5). By solving this equation, the diffraction angle when the wavelength component having the center wavelength $\lambda_0$ is incident on the dispersion member 44e at this incident angle is obtained as $\beta$.

Subsequently, the MFD obtained here, the incident angle in the dispersion member 44d, the incident angle in the dispersion member 44e, the diffraction angle in the dispersion member 44d, and the diffraction angle in the dispersion member 44e are substituted for $\omega_1$, $\alpha_1$, $\alpha_2$, $\beta_1$, and $\beta_2$, respectively, in Equation (10). Furthermore, $f_2/f_1$ is substituted for Power in Equation (10). Thus, the spot diameter of the wavelength component having the center wavelength $\lambda_0$ in the interference light condensed on the light receiving unit 15 is obtained in advance as $\omega_5$.

As described above, the average wavelength wl of the detection signal detected via the light receiving unit 15 decreases as the optical path length x from the zero point to the measurement target in the measurement arm (the difference in optical path length between the reference arm and the measurement arm) increases. Therefore, when the optical path length x from the zero point to the measurement target in the measurement arm becomes an assumed maximum value, the average wavelength wl takes an assumed minimum value.

In the present embodiment, the measurement targets are the corneal apex and retina of the eye to be examined. In the present embodiment, before measurement, the position of the corneal apex with respect to the SD-OCT device 1 is roughly a predetermined position by alignment via the alignment mechanism 13. Therefore, an individual difference in position of the corneal apex with respect to the SD-OCT device 1 is minute.

Furthermore, in a case where the measurement target is the retina, the control unit 10 drives the mirror via the adjustment mechanism 11 to adjust the position of the zero point in the measurement arm to be a position at a predetermined distance behind the cornea. The eye axis length (distance between the corneal apex and the retina) in the eyeball is individually different, and varies depending on each eyeball. Therefore, there may be individual differences in position of the retina with respect to the SD-OCT device 1. That is, there is individual differences in position assumed as the position of the retina of the eye to be examined on the optical path 43, and there is variation in the optical path length x from the zero point to the measurement target in the measurement arm. The magnitude of the variation in optical path length x is a value obtained by multiplying a value defined as the individual difference in eye axis length (a difference between the maximum value and the minimum value of the assumed eye axis length) by the refractive index of the vitreous body.

In the present embodiment, the individual difference in eye axis length is obtained from Reference Document 1 below.

Reference Document 1: C McAlinden, "Axial Length Measurement Failure Rates With Biometers Using Swept-Source Optical Coherence Tomography Compared to Partial-Coherence Interferometry and Optical Low-Coherence Interferometry", AMERICAN JOURNAL OF OPHTHALMOLOGY, Elsevier, 2017

In Reference Document 1, the assumed maximum value of the eye axis length is 26.56 mm, and the assumed minimum value of the eye axis length is 20.36 mm. Therefore, 26.56 mm - 20.36 mm = 6.2 mm is defined as the individual difference in eye axis length. The magnitude of the variation that can be caused in the optical path length x according to the individual difference in eye axis length is 8.2832 mm, which is a value obtained by multiplying this value (6.2 mm) by the refractive index (1.336) of the vitreous body.

As described above, in the SD-OCT device 1 that measures the corneal apex and the retina, it is assumed that a variation having a magnitude of 8.2832 mm may be caused in optical path length x according to the individual difference in eye axis length. Therefore, in the present embodiment, assuming that the optical path length x varies in a range up to 8.2832 mm, a range of from 0 mm to 8.2832 mm is assumed as the range of the optical path length x. Therefore, in the present embodiment, the assumed maximum value of the optical path length x is 8.2832 mm, which is the magnitude of the variation corresponding to the individual difference in eye axis length. Assuming that the optical path length x = 8.2832 mm, the minimum value assumed as the average wavelength of the detection signal is obtained in advance as wl using Equation (3) from the optical path length x, the minimum wavelength $\lambda_1$ and the maximum wavelength $\lambda_2$ among the wavelengths of the light that can be received by the light receiving unit 15, and the length L of the light receiving unit 15.

The SD-OCT device 1 of the present embodiment is designed such that the spot diameter cos of the wavelength component of the center wavelength $\lambda_0$ obtained in advance is equal to or less than the minimum value wl assumed as the average wavelength of the detection signal obtained in advance, that is, so as to satisfy a relationship according to Equation (11) below.

[Mathematical Formula 11]

$$\omega_1 \times (f_2/f_1) \times \prod_{i=1}^{n}(cos(\alpha_i)/cos(\beta_i)) \leq L/(2|x|(1/\lambda_1 - 1/\lambda_2)) \qquad (1\,1)$$

In the present embodiment, the transmission unit 44a, the dispersion member 44d, the dispersion member 44e, the lens 44f, and the light receiving unit 15 used in the SD-OCT device 1 are determined in advance. Further, a positional relationship among the dispersion member 44d, the dispersion member 44e, the lens 44f, and the light receiving unit 15 is also determined in advance. Therefore, parameters other than fi in Equation (11) are obtained from the transmission unit 44a, the dispersion member 44d, the dispersion member 44e, the lens 44f, the light receiving unit 15, and the positional relationship. Therefore, in designing the SD-OCT device 1 of the present embodiment, a lens having the focal length fi, which satisfies Equation (11), is selected as the lens 44c. As a result, in the SD-OCT device 1, the spot diameter cos of the wavelength component having the center wavelength $\lambda_0$ can be set to be equal to or less than the minimum value wl assumed as the average wavelength of the detection signal.

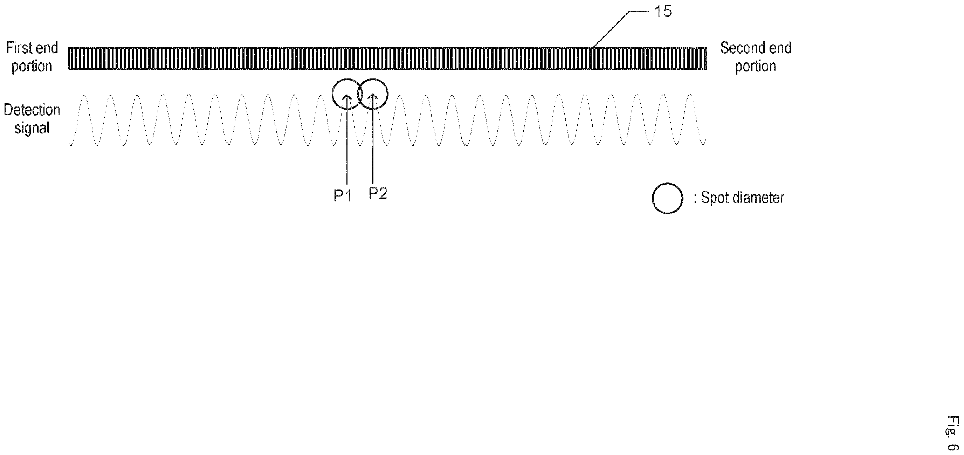

Here, an intention of such design that the spot diameter cos of the wavelength component having the center wavelength $\lambda_0$ is set to be equal to or less than the minimum value wl assumed as the average wavelength of the detection signal will be described with reference to FIG. 6.

Each of the wavelength components of the interference light is condensed in a finite size on the light receiving unit 15. Therefore, the wavelength component corresponding to a peak portion and the wavelength component corresponding to the adjacent peak portion in the detection signal detected via the light receiving unit 15 (for example, wavelength components corresponding to a peak P1 and a peak P2 in FIG. 6) are also condensed in a finite size on the light receiving unit 15. Circles around the peaks P1 and P2 in FIG. 6 indicate sizes of the spot diameters of the wavelength components in this case. As in the example in FIG. 6, these wavelength components are likely to at least partially overlap with each other on the light receiving unit 15. In particular, as the optical path length x from the zero point to the measurement target in the measurement arm increases, the average wavelength of the detection signal decreases, and the distance between the peaks in the detection signal decreases, so that the wavelength components corresponding to the adjacent peaks are more likely to overlap with each other on the light receiving unit 15. As described above, when the different wavelength components corresponding to the adjacent peaks of the detection signal overlap with each other, the signals of the wavelength components cannot be distinguished from each other at the overlapping portion, whereby the accuracy of detection of the intensity for each of the wavelength components via the light receiving unit 15 decreases. As a result, the accuracy of measurement of the position of the measurement target by the SD-OCT also decreases.

Therefore, in the present embodiment, the spot diameter of the wavelength component of the predetermined wavelength (center wavelength $\lambda_0$) condensed on the light receiving unit 15 is set to be equal to or less than the minimum value assumed as the average wavelength of the detection signal detected via the light receiving unit 15, thereby reducing the overlap between the wavelength components corresponding to the peaks of the detection signal.

As described above, according to the configuration of the SD-OCT device 1 of the present embodiment, the spot diameter of the wavelength component of the predetermined wavelength in the interference light condensed on the light receiving unit 15 can be set to be equal to or less than the average wavelength of the detection signal. Thus, in the SD-OCT device 1, the overlap between the signals corresponding to the adjacent peaks in the detection signal is reduced, and the decrease in accuracy in the measurement by the SD-OCT can be suppressed.

Further, in the present embodiment, the SD-OCT device 1 is designed such that the spot diameter of the wavelength component having the center wavelength $\lambda_0$ as the predetermined wavelength is equal to or less than the average wavelength of the detection signal. As a result, it is possible to reduce the overlap of the wavelength component of the wavelength mainly contributing to the measurement with other wavelength components, and also to further suppress the decrease in accuracy in the measurement by the SD-OCT.

Furthermore, in the present embodiment, the SD-OCT device 1 is designed such that the spot diameter of the wavelength component of the predetermined wavelength in the interference light condensed on the light receiving unit 15 is equal to or less than the minimum value assumed as the average wavelength of the detection signal. In the present embodiment, the measurement target includes the retina of the eyeball, and when the measurement target is the retina, the position of the retina varies due to individual differences. In the SD-OCT, when there is no measurement target in a range where good measurement is possible, the reference arm may be adjusted, the zero point in the measurement arm may be moved, and the range where good measurement is possible may be moved. That is, it may take time and effort to adjust the reference arm. As described above, in the SD-OCT, there is a possibility that it may take time and effort for a measurement target individually different in position. In the present embodiment, the spot diameter of the wavelength component of the predetermined wavelength in the interference light condensed on the light receiving unit 15 is equal to or less than the average wavelength of the detection signal as long as the position of the measurement target is within an assumed variation range. Therefore, when the position of the measurement target is within the assumed variation range, the overlap between the wavelength components corresponding to the peaks in the detection signal is reduced, whereby the decrease in accuracy in the measurement by the SD-OCT can be suppressed. As a result, the SD-OCT device 1 can improve the possibility of being able to perform measurement with high accuracy within the assumed variation range of the position of the measurement target, and can reduce the possibility of taking time and effort to adjust the reference arm.

(2) Eye axis length measurement processing:

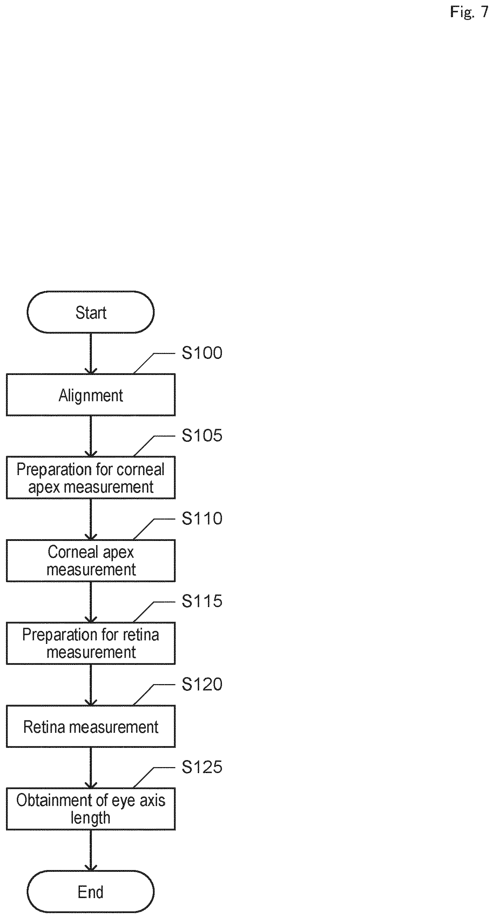

The eye axis length measurement processing executed by the SD-OCT device 1 of the present embodiment will be described with reference to FIG. 7. The control unit 10 starts the processing in FIG. 7 at a designated timing after the eye to be eye to be examined is placed at a predetermined position.

In step S100, the control unit 10 detects a position of the corneal apex of the eye to be examined of the subject present at a predetermined position via the alignment mechanism 13, and adjusts a position of the SD-OCT device 1 such that the detected position of the corneal apex and the SD-OCT device 1 have a predetermined positional relationship. After completion of the processing in step S100, the control unit 10 advances the processing to step S105.

In step S105, the control unit 10 adjusts the reference arm to serve as the reference arm for the cornea by moving the mirror 12 via the adjustment mechanism 11. After completion of the processing in step S105, the control unit 10 advances the processing to step S110.

In step S110, the control unit 10 causes the light source 14 to output light, and detects the intensity of the interference light for each of the wavelength components via the light receiving unit 15. The control unit 10 specifies the position of the corneal apex of the eye to be examined in the optical path 43 based on the intensity of the interference light detected for each of the wavelength components. After completion of the processing in step S110, the control unit 10 advances the processing to step S115.

In step S115, the control unit 10 adjusts the reference arm to serve as the reference arm for the reference arm for the retina by moving the mirror 12 via the adjustment mechanism 11. In the present embodiment, the spot diameter of the wavelength component of the predetermined wavelength in the interference light condensed on the light receiving unit 15 is equal to or less than the average wavelength of the detection signal as long as the position of the measurement target is within an assumed variation range, as described above. Therefore, when the position of the measurement target is within the assumed variation range, the overlap between the wavelength components corresponding to the peaks in the detection signal is reduced. That is, since the measurement target can be measured with sufficient accuracy within the assumed variation range, adjustment of the reference arm is not required. Therefore, in the present embodiment, the control unit 10 adjusts the reference arm to serve as a reference arm for the retina via the adjustment mechanism 11, and then performs control so as not to adjust the reference arm via the adjustment mechanism 11, that is, so as not to adjust an optical path length of the reference light until the position of the retina is specified. As a result, the control unit 10 can reduce the burden on the processing without performing unnecessary processing via the adjustment mechanism 11. After completion of the processing in step S115, the control unit 10 advances the processing to step S120.

In step S120, the control unit 10 causes the light source 14 to output light, and detects the intensity of the interference light for each of the wavelength components via the light receiving unit 15. The control unit 10 specifies the position of the retina of the eye to be examined in the optical path 43 based on the intensity of the interference light detected for each of the wavelength components.

In step S125, the control unit 10 acquires, as the eye axis length, a difference between the position of the retina specified in step S120 and the position of the corneal apex specified in step S110.

(3) Other embodiments:

The above embodiment is an example for carrying out the present disclosure, and various other embodiments can also be adopted. Therefore, at least a part of the configuration of the embodiment described above may be omitted, or replaced.

In the embodiment described above, the SD-OCT device 1 is designed such that the spot diameter, in the light receiving unit 15, of the wavelength component having the center wavelength $\lambda_0$ as the wavelength component having the predetermined wavelength in the interference light is equal to or less than the average wavelength of the detection signal. However, the predetermined wavelength here may be a wavelength different from the center wavelength $\lambda_0$.

For example, the predetermined wavelength may be the maximum wavelength $\lambda_2$ among wavelengths of light that can be received by the light receiving unit 15. In this case, the SD-OCT device 1 is designed such that the spot diameter, in the light receiving unit 15, of the wavelength component having the wavelength $\lambda_2$ in the interference light is equal to or less than the average wavelength of the detection signal. This spot diameter is obtained, for example, as follows.

The MFD of the wavelength component of the wavelength $\lambda_2$ output from the transmission unit 44a is obtained from the wavelength $\lambda_2$, a core diameter of the transmission unit 44a, and the refractive indexes of the core and the clad of the transmission unit 44a. In addition, the incident angle and the diffraction angle when the wavelength component having the wavelength $\lambda_2$ is incident on the dispersion member 44d are obtained by the same method as in the embodiment described above. Similarly, the incident angle and the diffraction angle when the wavelength component having the wavelength $\lambda_2$ is incident on the dispersion member 44e are obtained. The MFD obtained here, the incident angle in the dispersion member 44d, the incident angle in the dispersion member 44e, the diffraction angle in the dispersion member 44d, and the diffraction angle in the dispersion member 44e are substituted for $\omega_1$, $\alpha_1$, $\alpha_2$, $\beta_1$, and $\beta_2$, respectively, in Equation (10). Furthermore, $f_2/f_1$ is substituted for Power in Equation (10). Thus, the spot diameter of the wavelength component having the wavelength $\lambda_2$ in the interference light condensed on the light receiving unit 15 is obtained as $\omega_5$.

The diffraction angle $\beta$ increases as the wavelength of the light incident on the diffraction grating increases. Therefore, as the wavelength of the light is larger, cos ($\beta$i) in Equation (10) is smaller, and (cos ($\alpha_i$)/cos ($\beta_i$)) in Equation (10) is larger. Therefore, (cos ($\alpha_i$)/cos ($\beta_i$)) in Equation (10) is the largest at the wavelength $\lambda_2$.

Furthermore, the MFD is also defined as Equation (12)below. $\lambda$ in Equation (12) represents a wavelength of light transmitted through the optical fiber. In addition, $\theta$ represents a radiation angle of light formed with a propagation axis of the optical fiber. F($\theta$) represents an electric field distribution of a far field pattern (FFP).

[Mathematical Formula 12]

$$MFD = (\lambda/\pi)\left[\frac{\int_0^{\pi/2} F^2(\theta)\sin\theta\,\cos\theta d\theta}{\int_0^{\pi/2} F^2(\theta)\sin^3\theta\,\cos\theta d\theta}\right]^{1/2} \qquad (12)$$

As shown in Equation (12), the MFD increases as the wavelength ($\lambda$) of light increases. That is, the MFD for the wavelength component having the wavelength $\lambda_2$ is larger than the MFD for the wavelength component having the wavelength of $\lambda_2$ or less. Therefore, $\omega_1$ in Equation (10) is the largest at the wavelength $\lambda_2$.

As described above, the spot diameter for the wavelength $\lambda_2$ is the largest among the spot diameters of the wavelength components condensed on the light receiving unit 15.

Therefore, by virtue of such design that the spot diameter of the wavelength component having the wavelength

$\lambda_2$ in the light receiving unit 15 is equal to or less than the average wavelength of the detection signal, the spot diameters of the wavelength components corresponding to all the peaks in the detection signal become equal to or less than the average wavelength of the detection signal. That is, the wavelength components corresponding to all the adjacent peaks in the detection signal do not overlap with each other on the light receiving unit 15. Thus, the SD-OCT device 1 can further suppress a decrease in accuracy of the measurement by the SD-OCT.

Also, the predetermined wavelength may be the minimum wavelength $\lambda_1$ among the wavelengths of light that can be received by the light receiving unit 15. In this case, the SD-OCT device 1 is designed such that the spot diameter, in the light receiving unit 15, of the wavelength component having the wavelength $\lambda_1$ in the interference light is equal to or less than the average wavelength of the detection signal.

In the embodiment described above, the center wavelength $\lambda_0$ is the center wavelength in the wavelength band of the light output from the light source 14. However, the center wavelength $\lambda_0$ may be another wavelength. For example, the center wavelength $\lambda_0$ is a wavelength near the center of the wavelength band of the light output from the light source 14, and may be a wavelength different from the center wavelength of this wavelength band. For example, the center wavelength $\lambda_0$ may be any wavelength within a range of a predetermined width (for example, 5% of the entire wavelength band) at the center of the wavelength band of the light output from the light source 14, and may be a wavelength different from the center wavelength of this wavelength band. The center wavelength $\lambda_0$ may be a wavelength of light received by a portion of the central light receiving element among the plurality of linearly arranged light receiving elements included in the light receiving unit 15. Furthermore, the center wavelength $\lambda_0$ may be an average value of the maximum value $\lambda_2$ and the minimum value $\lambda_1$ of the wavelengths of the light received by the light receiving unit 15.

In the embodiment described above, the individual difference in assumed eye axis length is 6.2 mm, which is a value determined from Reference Literature 1. However, the individual difference in assumed eye axis length may be another value.

For example, the individual difference in assumed eye axis length may be a value obtained from Reference Document 2 below.

Reference Document 2: J. Jonas, "Retinal Thickness and Axial Length", IOVS, the Association for Research in Vision and Ophthalmology (ARVO), 2016

In Reference Document 2, the assumed maximum value of the eye axis length is 28.68 mm, and the assumed minimum value of the eye axis length is 20.29 mm. Therefore, the individual difference in eye axis length obtained from Reference Document 2 is 28.68 mm - 20.29 mm = 8.39 mm. In the measurement arm, the individual difference in eye axis length may cause a variation in optical path length of 11.20904 mm, which is a value obtained by multiplying the individual difference (8.39 mm) by the refractive index (1.336) of the vitreous body. Therefore, a range of 0 mm to 11.20904 mm is assumed as the variation range of the optical path length x. The assumed maximum value of the optical path length x from the zero point to the measurement target in the measurement arm is 11.20904 mm. Assuming that the optical path length x = 11.20904 mm, the minimum value assumed as the average wavelength of the detection signal is obtained in advance as wl using Equation (3) from the optical path length x, the minimum wavelength $\lambda_1$ and the maximum wavelength $\lambda_2$ among the wavelengths of the light that can be received by the light receiving unit 15, and the length L of the light receiving unit 15. Then, the SD-OCT device 1 may be designed such that the spot diameter of the wavelength component having the predetermined wavelength in the interference light is equal to or less than wl obtained here.

Also, the individual difference in assumed eye axis length may be a value obtained from Reference Document 3 below.

Reference Document 3: Markus Kohlhaas, "Effect of Central Corneal Thickness, Corneal Curvature, and Axial Length on Applanation Tonometry", American Medical Association, 2006

In Reference Document 3, the assumed maximum value of the eye axis length is 32.93 mm, and the assumed minimum value of the eye axis length is 18.84 mm. Therefore, the individual difference in eye axis length obtained from Reference Document 3 is 32.93 mm - 18.84 mm = 14.09 mm. In this case, 18.82424 mm, which is a value obtained by multiplying this value by the refractive index (1.336) of the vitreous body, is obtained as the magnitude of the variation in optical path length from the zero point to the measurement target in the measurement arm. Here, it is assumed that the optical path length x varies in a range of 0 mm to 18.82424 mm. In this case, assuming that the optical path length x = 18.82424 mm, the minimum value assumed as the average wavelength of the detection signal is obtained in advance as wl using Equation (3) from the optical path length x, the minimum wavelength $\lambda_1$ and the maximum wavelength $\lambda_2$ among the wavelengths of the light that can be received by the light receiving unit 15, and the length L of the light receiving unit 15. Then, the SD-OCT device 1 may be designed such that the spot diameter of the wavelength component having the predetermined wavelength in the interference light is equal to or less than wl obtained here.

Furthermore, in the embodiment described above, the SD-OCT device 1 is designed such that the spot diameter of the wavelength component having the predetermined wavelength in the interference light is equal to or less than the average wavelength wl of the detection signal in a case where the optical path length x takes the assumed maximum value. However, the SD-OCT device 1 may be designed such that the spot diameter of the wavelength component having the predetermined wavelength in the interference light is equal to or less than the average wavelength wl obtained from Equation (3) in a case where the optical path length x takes a value different from the assumed maximum value (for example, a median value of the assumed variation in optical path length x, or an arbitrary value in a range of the assumed variation in optical path length x).

Furthermore, in the embodiment described above, the control unit 10 adjusts the reference arm by moving the mirror 12 via the adjustment mechanism 11. However, the control unit 10 may adjust the reference arm by another method. For example, it is assumed that a plurality of optical systems forming a plurality of optical paths having different optical path lengths are prepared as optical paths at an output end *et seq.* of the transmission unit 42a in the optical path 42. Then, it is assumed that a rotary mirror for changing the traveling direction of the light traveling through the optical path 42 to any of these optical systems is provided. In this case, for example, the adjustment mechanism 11 is a mechanism that rotates the rotary mirror. The control unit 10 may adjust the reference arm by adjusting an angle of the rotary mirror provided on the optical path 42 via the adjustment mechanism 11 and advancing the reference light to any of the plurality of optical systems.

Furthermore, in the embodiment described above, the spot diameter of the wavelength component of the predetermined wavelength condensed on the light receiving unit 15 is obtained using Equation (10). However, the spot diameter of the wavelength component of the predetermined wavelength condensed on the light receiving unit 15 may be obtained by another method. For example, by applying an optical filter to the interference light output from the transmission unit 44a and shielding wavelength components other than the wavelength component having the predetermined wavelength, only the wavelength component having the predetermined wavelength is condensed on the light receiving unit 15. Then, the spot diameter of the wavelength component of the predetermined wavelength condensed on the light receiving unit 15 may be obtained by measuring the diameter of the wavelength component condensed on the light receiving unit 15. In this case, the SD-OCT device 1 may be designed such that the measured spot diameter is equal to or less than the average wavelength of the detection signal.

Furthermore, in the embodiment described above, the SD-OCT device 1 is designed by selecting the lens 44c so that the spot diameter having the predetermined wavelength component having the interference light is equal to or less than the average wavelength of the detection signal. However, the SD-OCT device 1 may be designed by selecting a member different from the lens 44c. For example, in a case where each element constituting the optical system 44b is determined in advance, a parameter other than $\omega_1$ in Equation (11) is predetermined. In this case, the optical fiber constituting the transmission unit 44a may be selected such that the MFD for the predetermined wavelength is $\omega_1$ that satisfies Equation (11). Furthermore, the SD-OCT device 1 may be designed by selecting a plurality of elements among the elements constituting the optical path 44.

In addition, in the embodiment described above, the measurement targets are the corneal apex and retina of the eye to be examined. However, the measurement targets may be other objects such as other sites (sites different from the corneal apex in the cornea, iris, conjunctiva, and the like) of the eye to be examined.

Furthermore, in the embodiment described above, the number n of the dispersion members included in the optical system 44b is 2. However, n may be 1 or 3 or more.

In addition, in the embodiment described above, the SD-OCT device 1 includes the Michelson interferometer configured as the interferometer that generates the interference light. However, the SD-OCT device 1 may include another interferometer such as a balanced Michelson interferometer or a Mach-Zehnder interferometer.

The value defined as the individual difference that can be caused for the eye axis length of the eyeball may be any value defined as the individual difference in eye axis length based on measurement results of eye axis lengths of a plurality of eyeballs. For example, the value may be a difference between a minimum value and a maximum value of actually measured eye axis lengths of eyeballs of a plurality of persons.

**Claims**

1. An SD-OCT device comprising:

   a light source (14) configured to output light including a plurality of wavelength components;
   a branching unit (30) configured to branch, from the light output from the light source (14), at least reference light following a reference optical path and measurement light applied to a measurement target;
   a transmission unit (44a) configured to transmit interference light between the reference light and the measurement light returning from the measurement target;
   a light receiving unit (15) in which a plurality of light receiving elements are linearly arranged; and
   an optical system (44b) configured to disperse the interference light output from the transmission unit (44a) and condenses the interference light on the light receiving unit (15) for each of the wavelength components,
   wherein a diameter of the wavelength component having a predetermined wavelength, which is condensed on the light receiving unit (15) by the optical system (44b), is equal to or less than an average wavelength of a wavy signal detected via the light receiving unit (15) upon reception of the interference light.

2. The SD-OCT device according to claim 1, wherein
the predetermined wavelength is a center wavelength of the light.

3. The SD-OCT device according to claim 1, wherein
the predetermined wavelength is a maximum wavelength among wavelengths of the wavelength components that can be received by the light receiving unit (15).

4. The SD-OCT device according to claim 1, 2 or 3, wherein

the transmission unit (44a) is an optical fiber,
the optical system (44b) comprises one or more dispersion members, and
the diameter of the wavelength component having the predetermined wavelength, which is condensed on the light receiving unit (15) by the optical system (44b), is a value obtained from Equation (1), based on a Mode Field Diameter (MFD) when the wavelength component having the predetermined wavelength is output from the transmission unit (44a), a magnification Power of the optical system (44b), a number n of the dispersion members included in the optical system (44b), and incident angles $\alpha_1$ to $\alpha_n$ and diffraction angles $\beta_1$ to $\beta_n$ in a case where the wavelength component having the predetermined wavelength passes through each of the n dispersion members
[Mathematical Formula 1]

$$MFD \times Power \times \prod_{i=1}^{n}(cos(\alpha_i)/cos(\beta_i)) \qquad (1)$$

5. The SD-OCT device according to claim 4, wherein
the interference light incident on the dispersion members is parallel light.

6. The SD-OCT device according to any of the preceding claims, wherein

the measurement target is a retina, and
the average wavelength is a value obtained from Equation (2), based on an optical path length x corresponding to a value defined as an individual difference that can be caused with respect to an eye axis length of an eyeball, a minimum wavelength $\lambda_1$ and a maximum wavelength $\lambda_2$ among a plurality of wavelengths corresponding to a plurality of the wavelength components received by the light receiving unit (15), and a length L of the light receiving unit (15)
[Mathematical Formula 2]

$$L/(2|x|(1/\lambda_1 - 1/\lambda_2)) \qquad (2)$$

7. The SD-OCT device according to claim 6, wherein
the optical path length x is any one of 8.2832 mm, 11.20904 mm, and 18.82424 mm.

8. The SD-OCT device according to any of the preceding claims, further comprising:

an adjustment mechanism (11) configured to adjust an optical path length of the reference light; and
a control unit (10) configured to adjust the optical path length of the reference light using the adjustment mechanism (11) for measurement of a predetermined object in a case where the predetermined object is measured as the measurement target, and configured to perform control so as not to adjust the optical path length of the reference light using the adjustment mechanism (11) during the measurement of the predetermined object.

Fig. 1

Fig. 2

Fig. 3

44d

44e

44c

60°

44f

44a

Second end
portion

First end
portion

15

Fig. 4

Fig. 5

Fig. 6

Second end portion

15

First end portion

Detection signal

P1 P2

◯ : Spot diameter

Fig. 7

```
        ┌─────────────┐
        │    Start    │
        └──────┬──────┘
               │         ┌─ S100
               ▼
        ┌─────────────┐
        │  Alignment  │
        └──────┬──────┘
               │         ┌─ S105
               ▼
    ┌────────────────────┐
    │ Preparation for    │
    │ corneal apex       │
    │ measurement        │
    └──────┬─────────────┘
               │         ┌─ S110
               ▼
    ┌────────────────────┐
    │  Corneal apex      │
    │  measurement       │
    └──────┬─────────────┘
               │         ┌─ S115
               ▼
    ┌────────────────────┐
    │ Preparation for    │
    │ retina measurement │
    └──────┬─────────────┘
               │         ┌─ S120
               ▼
    ┌────────────────────┐
    │ Retina measurement │
    └──────┬─────────────┘
               │         ┌─ S125
               ▼
    ┌────────────────────┐
    │ Obtainment of eye  │
    │ axis length        │
    └──────┬─────────────┘
               │
               ▼
        ┌─────────────┐
        │     End     │
        └─────────────┘
```

Europäisches
Patentamt

European
Patent Office

Office européen
des brevets

## EUROPEAN SEARCH REPORT

Application Number

EP 22 20 7401

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/073655 A1 (CANON KK [JP]; KOBAYASHI SHUICHI [JP]) 1 July 2010 (2010-07-01) * paragraph [0015] – paragraph [0021] * * paragraph [0043] * * paragraph [0056] * * paragraph [0059] * * figures 1A-B, 2, 3A, 3C-D 7A-B * | 1-8 | INV. A61B3/10 |
| A | WO 2016/187675 A1 (CYLITE PTY LTD [AU]) 1 December 2016 (2016-12-01) * page 19, line 27 – page 22, line 10 * * figures 4, 5, 11, 12 * | 1-8 | |

TECHNICAL FIELDS
SEARCHED       (IPC)

A61B

The present search report has been drawn up for all claims

| Place of search | Date of completion of the search | Examiner |
|---|---|---|
| Munich | 23 March 2023 | Horváth, László |

EPO FORM 1503 03.82 (P04C01)

## EP 4 183 321 A1

### ANNEX TO THE EUROPEAN SEARCH REPORT
### ON EUROPEAN PATENT APPLICATION NO.

EP 22 20 7401

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2023

| Patent document cited in search report | Publication date | Patent family member(s) | Publication date |
|---|---|---|---|
| WO 2010073655 A1 | 01-07-2010 | BR PI0923709 A2 | 19-01-2016 |
| | | CN 102264282 A | 30-11-2011 |
| | | EP 2385783 A1 | 16-11-2011 |
| | | JP 5618533 B2 | 05-11-2014 |
| | | JP 5901716 B2 | 13-04-2016 |
| | | JP 2010167268 A | 05-08-2010 |
| | | JP 2014231022 A | 11-12-2014 |
| | | KR 20110106391 A | 28-09-2011 |
| | | KR 20130042658 A | 26-04-2013 |
| | | RU 2011131064 A | 10-02-2013 |
| | | RU 2013114869 A | 10-10-2014 |
| | | US 2011228222 A1 | 22-09-2011 |
| | | WO 2010073655 A1 | 01-07-2010 |
| WO 2016187675 A1 | 01-12-2016 | AU 2016267409 A1 | 18-01-2018 |
| | | CN 107615005 A | 19-01-2018 |
| | | CN 113180589 A | 30-07-2021 |
| | | EP 3303985 A1 | 11-04-2018 |
| | | JP 6909207 B2 | 28-07-2021 |
| | | JP 2018516375 A | 21-06-2018 |
| | | JP 2021168929 A | 28-10-2021 |
| | | US 2016345820 A1 | 01-12-2016 |
| | | US 2018228363 A1 | 16-08-2018 |
| | | US 2020170501 A1 | 04-06-2020 |
| | | WO 2016187675 A1 | 01-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021186837 A
- JP 2016032578 A

### Non-patent literature cited in the description

- Axial Length Measurement Failure Rates With Biometers Using Swept-Source Optical Coherence Tomography Compared to Partial-Coherence Interferometry and Optical Low-Coherence Interferometry. **C MCALINDEN.** AMERICAN JOURNAL OF OPHTHALMOLOGY. Elsevier, 2017
- **J. JONAS.** Retinal Thickness and Axial Length. *IOVS, the Association for Research in Vision and Ophthalmology (ARVO),* 2016
- **MARKUS KOHLHAAS.** Effect of Central Corneal Thickness, Corneal Curvature, and Axial Length on Applanation Tonometry. American Medical Association, 2006